# EUROPÄISCHE PATENTSCHRIFT

(11) **EP 2 467 603 B1**
(45) Veröffentlichungstag und Bekanntmachung des Hinweises auf die Patenterteilung: **15.05.2013**
(21) Anmeldenummer: 10752295.5
(22) Anmeldetag: 17.08.2010
(51) Int. Cl.: F04B 43/02, F04B 43/04, A61M 1/10

(54) **EINWEGELEMENT, SYSTEM ZUM PUMPEN SOWIE VERFAHREN ZUM PUMPEN EINER FLÜSSIGKEIT**
DISPOSABLE ELEMENT, SYSTEM FOR PUMPING AND METHOD FOR PUMPING A LIQUID
ELÉMENT À USAGE UNIQUE, SYSTÈME DE POMPAGE AINSI QUE PROCÉDÉ DE POMPAGE D UN LIQUIDE

(30) Priorität: 18.08.2009 DE 102009037845
(43) Veröffentlichungstag der Anmeldung: 27.06.2012
(73) Patentinhaber: Fresenius Medical Care Deutschland GmbH, 61352 Bad Homburg (DE)
(72) Erfinder: ÖRTER, Gökhan, 35789 Weilmünster (DE)
(74) Vertreter: Laufhütte, Dieter
(86) Internationale Anmeldenummer: PCT/EP2010/005064
(87) Internationale Veröffentlichungsnummer: WO 2011/020600

(56) Entgegenhaltungen:
- DE-A1- 4 118 628
- DE-C- 589 398
- US-A- 5 011 380
- US-A1- 2002 098 122
- US-A1- 2004 265 150

## Beschreibung

Die vorliegende Erfindung betrifft ein Einwegelement bestehend aus zumindest einem ersten Teil, in welchem in der Oberfläche Kanalstrukturen ausgenommen sind, und einem dieses dichtend abdeckenden zweiten Teil, wobei das erste Teil und/oder das zweite Teil zum größten Teil starr ausgebildet und/oder auf einer starren Trägerstruktur aufgebracht ist/sind und wobei diese ersten und/oder zweiten Teile jedoch zumindest einen flexibel ausgebildeten Bereich aufweisen. Des Weiteren betrifft die Erfindung ein System zum Pumpen sowie ein Verfahren zum Pumpen einer Flüssigkeit.

Aus dem Stand der Technik sind bereits Einwegkassetten bekannt, die vorzugsweise im Bereich der Analysentechnik eingesetzt werden. Diese Kassetten weisen beispielsweise mehrere Layer auf, die starre und flexible Bereiche aufweisen und unter Verwendung der Zweikomponentenspritzgusstechnologie einstückig hergestellt werden. Die flexiblen Bereiche können beispielsweise als Pumpkammern ausgeführt sein, die durch einen entsprechenden Aktor eingedrückt werden können, so dass eine Fluidverdrängung stattfindet. Eine Pumpbewegung und/oder auch entsprechende Ventilfunktionen in den flexiblen Bereichen werden somit durch mechanisch angetriebene Stößel erzeugt. Solche mechanischen Schnittstellen erzeugen jedoch Probleme bei der Ankopplung der Einwegkassetten an eine entsprechende Maschine. Zum Einen kann das sogenannte Disposable nicht vollständig glatt an das Maschinenteil angelegt werden, da immer Vorsprünge oder Ausnehmungen der mechanischen Schnittstellen bestehen. Zum Anderen kann eine mechanische Aktivierung der Membranen Beschädigungen hervorrufen, etwa durch die mechanischen Walkbewegungen der flexiblen Bereiche, was zum Versagen des Bauteils führen kann. Eine derartige, entsprechende Einwegkassette ist beispielsweise aus der DE 102 39 597 A1 bekannt.

Weiter beschreibt die US 6,261,065 B1 ein Blutbehandlungssystem, das an eine Bluttrennvorrichtung angeschlossen ist. Dieses System weist eine Kassette auf, die wenigstens eine pneumatisch betätigte Pumpe aufweist.

Die US 2007/0278155 A1 beschreibt ein mechanisches Fluidsystem mit Einwegkassetten, die flexible Schichten aufweisen. Zur hochgenauen Flüssigkeitsbilanzierung sind Ausgleichskammersysteme beschrieben, die eine erste und eine zweite Pumpkammer aufweisen und durch eine flexible Membran getrennt sind. Durch das Einströmen einer ersten Fluidmenge in eine erste Kammer der Ausgleichskammer wird die entsprechende Menge an Fluid aus der zweiten Kammer verdrängt. Für diese Ausgleichspumpbewegung kann an der die beiden Kammern trennenden flexiblen Membran ein Permanentmagnet eingearbeitet sein, der durch externe Elektromagneten bewegt werden kann.

Aus der DE 58 93 98 ist weiter eine elektromagnetisch betriebene Membranpumpe bekannt, die insbesondere zum Durchlüften von Aquarien vorgesehen ist. Mittels eines Elektromagneten wird ein Magnetanker zum Schwingen gebracht, so dass ein Luftstoß erzeugbar ist, der mittels eines Druckrohres beispielsweise in das Aquarium eingeleitet werden kann.

Aus der DE 199 63 306 A1 ist ein Antrieb für Blutpumpen bekannt, der in Form eines Lautsprecherantriebs ausgebildet ist. Dabei weist der Antrieb ein Magnetsystem und einen durch Absolut-Weg-Aufnehmer kontrollierten, mechanisch gleitenden Kolben mit um- und/oder abschaltbaren Wicklungen auf, bei welchem der Kolben mechanisch festlegbar ist.

Die US 4,459,977 betrifft eine Vorrichtung zur diastolischen Blutretroperfusion, die eine elektromagnetisch betriebene Membranpumpe aufweisen kann.

Die US 4,498,850 beschreibt eine Pumpe mit einer durch eine magnetisch aktivierbare Membran in zwei Kammern abgeteilte Pumpkammerausnehmung, die in einem Pumpkammergehäuse eingelassen ist. Um die Ausnehmung herum sind mehrere Mittel vorgesehen, mittels derer ein magnetisches Feld angelegt wird, so dass die magnetisch aktivierbare Membran bewegt werden kann, um eine Pumpbewegung auszulösen.

Die WO 2006/123329 A2 beschreibt eine Abgabevorrichtung für ein therapeutisches Fluid, insbesondere eine Abgabevorrichtung für Insulin, die handlich und vorzugsweise im Scheckkartenformat ausgeführt ist. Im Zusammenhang mit dieser Vorrichtung ist eine Membranpumpe beschrieben, die mit zwei Membranventilen, die jeweils vorgeschaltet sind und ebenfalls elektromagnetisch aktiviert werden, zusammenwirkt.

Die US 5,011,380 offenbart eine Verdrängerpumpe mit einer Membran und einen darauf aufgesetzten Magneten. Die Membran steht mit einem starren Träger über einen Klemmmechanismus in Verbindung. Der Gegenstand des Patentanspruchs 1 ist in zweiteiliger Form gegenüber dieser Druckschrift abgegrenzt.

Aus der US 2002/0098122 A1 ist eine Membranpumpe bekannt, deren Pumpbewegung durch magnetische Wechselwirkung erzeugt wird. Ein entsprechender Permanentmagnet ist auf der Oberfläche der Membran angeordnet und durch eine zusätzliche Schutzschicht abgedeckt.

Die US 2004/0265150 A1 offenbart eine Membran, bei der zu einer nichtmagnetischen Grundsubstanz magnetische Artikel beigemischt sind. Die Membran wird über einen Klemmmechanismus am Pumpgehäuse befestigt.

Aus der DE 41 18 628 A1 ist eine elektrische Membranpumpe bekannt, bei der die Membran sowie die Ventilplatte fest zwischen den jeweiligen Gehäuseabschnitten eingepresst ist.

Es ist daher die Aufgabe der vorliegenden Erfindung, ein Einwegelement der eingangs genannten Art in vorteilhafter Weise weiterzubilden, insbesondere dahingehend, dass dieses berührungslos betätigbar, einfach und sicher betreibbar sowie in der Lage ist, hochgenau Flüssigkeiten, insbesondere medizinische Flüssigkeiten zu pumpen.

Diese Aufgabe wird erfindungsgemäß gelöst durch ein Einwegelement mit den Merkmalen des Anspruchs 1. Danach ist vorgesehen, dass ein Einwegelement aus zumindest einem ersten Teil, in welchem in der Oberfläche Kanalstrukturen ausgenommen sind, und einem dieses dichtend abdeckenden zweiten Teil besteht, wobei das erste Teil und/oder das zweite Teil zum größten Teil starr ausgebildet und/oder auf einer starren Trägerstruktur aufgebracht ist/sind und wobei diese ersten und/oder zweiten Teile jedoch zumindest einen flexibel ausgebildeten Bereich aufweisen. Weiter ist vorgesehen, dass der wenigstens eine flexible Bereich zumindest einen Permanentmagneten und/oder zumindest einen permanentmagnetischen Bereich aufweist, so dass durch Anlegen eine Magnetfeldes mittels des flexiblen Bereichs Flüssigkeit verdrängbar ist.

Dadurch ergibt sich der Vorteil, dass zur Erzeugung der Bewegung des flexiblen Bereiches mit dem Permanentmagneten nunmehr keine mechanische Schnittstelle mit einem Maschinenteil, das den flexiblen Bereich antreibt, notwendig ist. Vielmehr ist es nunmehr möglich, berührungslos auf den flexiblen Bereich einzuwirken, um hierdurch Flüssigkeit verdrängen zu können. Aufgrund des Wegfalls der Stößel, die bislang auf entsprechende flexible Bereiche bekannter Einwegelemente wie Einwegkassetten eingewirkt haben, wird das Ankoppeln des erfindungsgemäßen Einwegelementes an eine entsprechende Maschine vereinfacht. Denn nunmehr ist ein im Wesentlichen glattes Anlegen an den entsprechenden Maschinenteil möglich, da aufgrund des Wegfalls der mechanischen, beweglichen Schnittstellenkomponenten nunmehr eine entsprechend glatte, angepasste Oberfläche vorgesehen werden kann. Darüber hinaus kann es aufgrund der berührungslosen Aktivierung nun nicht mehr zu mechanischen Beschädigungen kommen, was die Versagenswahrscheinlichkeit des Bauteils minimiert. Darüber hinaus ergibt sich der Vorteil, dass der Energieverbrauch zur Aktivierung einer Pumpe, die beispielsweise durch den flexiblen Bereich ausgebildet sein kann, reduziert werden kann. Weiter ergibt sich der Vorteil, dass die entsprechende Vorrichtung, in die das Einwegelement eingesetzt werden kann, nunmehr einfacher und kompakter ausgeführt werden kann.

Es ist denkbar, dass für den flexiblen Bereich gängige thermoplastische Elastomere verwendet werden. Vorzugsweise können Polymerblends aus SEBS und PP verwendet werden. Diese Mischungen weisen den Vorteil auf, dass diese dampfsterilisierbar sind. Alternativ ist denkbar, dass auch andere Mischungen Verwendung finden können, sofern diese mit den gängigen Sterilisationsmethoden in der Medizintechnik kompatibel sind. Es ist denkbar, dass der erste und der zweite Teil schichtartig ausgebildet sind. Auch die Trägerstruktur kann schichtartig ausgebildet sein, und beispielsweise die den als vollständig flexible Schicht ausgebildeten ersten Teil, in dem die Kanalstrukturen ausgebildet sind, tragen. In diesem Zusammenhang ist vorteilhaft denkbar, dass der erste Teil eine Mittelschicht bildet, die sandwichartig von der Trägerstruktur auf der einen Seite und auf der anderen Seite von der dichtend abdeckenden zweiten Schicht, die durch den zweiten Teil ausgebildet wird, umfasst ist.

Erfindungsgemäß ist das Einwegelement hergestellt durch ein Verfahren, in welchem starre und flexible Bereiche des ersten und/oder zweiten Teils mittels Zweikomponentenspritzguss einstückig hergestellt werden.

Ferner ist vorteilhaft denkbar, dass Trägerstruktur und/oder erstes und/oder zweites Teil mittels Zweikomponentenspritzguss einstückig hergestellt werden. Dadurch ergibt sich der Vorteil, kostengünstig und einfach in nur einem einzigen Spritzgussvorgang die vorteilhafte Struktur des Einwegelements mit starren und flexiblen Bereichen ausbilden zu können.

Des Weiteren kann vorgesehen sein, dass der wenigstens eine flexible Bereich mit dem Permanentmagnet und/oder dem permanentmagnetischen Bereich kammerartig und/oder domartig ausgebildet ist. In diesem Zusammenhang ist beispielsweise denkbar, dass der Kammerboden durch den zweiten Teil ausgebildet wird, der den ersten Teil dichtend abdeckt. Dadurch ergibt sich der Vorteil, dass eine fluiddynamisch günstige und zugleich einfach realisierbare Pumpkammer ausgebildet werden kann.

Von Vorteil ist es, wenn der kammerartig ausgebildete flexible Bereich eine Pumpkammer einer Membranpumpe ist.

Darüber hinaus kann vorgesehen sein, dass der flexible Bereich zumindest einen Zulauf und zumindest einen Ablauf aufweist, wobei Zulauf und Ablauf jeweils durch eine Kanalstruktur gebildet werden und/oder an eine Kanalstruktur angeschlossen sind. Denkbar ist in diesem Zusammenhang, dass im Bereich oder in funktionalem Zusammenhang mit Zulauf und Ablauf jeweils ein Ventilelement vorhanden ist, so dass der flexible Bereich als Membranpumpe nutzbar ist. So kann für die Ansaugphase der Pumpenkammer der Membranpumpe vorgesehen sein, dass das Ventilelement im Ablauf geschlossen und das Ventilelement im Zulauf geöffnet ist. Für die Auswurfphase wird sodann das Ventilelement im Zulauf geschlossen und im Ablauf geöffnet.

Erfindungsgemäß ist der Permanentmagnet und/oder der permanentmagnetische Bereich ein Einlegeteil , dass vollständig von dem flexiblen Bereich ummantelt wird. Dadurch ergibt sich der Vorteil, dass ein derartiges Einlegeteil vor dem Kunststoffspritzgießen insbesondere vor Beginn des Zweikomponentenspritzgusses in das Spritzgießwerkzeug eingelegt werden kann und sodann vollständig vom flexiblen Anteil des Einwegelements während der Fertigung ummantelt wird. Dadurch wird es möglich, den Permanentmagneten und/oder den permanentmagnetischen Bereich einfach in das Pumpsegment einzuarbeiten und weiter den Vorteil zu erzielen, dass der Permanentmagnet und/oder der permanentmagnetische Bereich aufgrund seiner Ummantelung keinen Fluidkontakt hat.

Ferner kann vorgesehen sein, dass der Permanentmagnet und/oder der permanentmagnetische Bereich in einem Teil des flexiblen Bereichs angeordnet ist, der einer Wandung gegenüberliegt, so dass durch Anlegen eines Magnetfeldes mit einer ersten Ausrichtung der flexible Bereich in Richtung der Wandung bewegbar ist und/oder dass durch Anlegen eines Magnetfeldes mit einer zweiten Ausrichtung der flexible Bereich von der Wandung weg bewegbar ist. Bei der Wandung kann es sich vorteilhafterweise um einen Bereich des zweiten Teils handeln. Denkbar ist dabei, dass bei Anlegen eines Magnetfeldes in der ersten Ausrichtung vorteilhafterweise eine Verringerung des Pumpkammervolumens erfolgt und bei Anlegen des Magnetfeldes mit der zweiten Ausrichtung eine Rückstellung der Kammerwandungen erfolgt und die Pumpkammer in diesem Schritt auch bereits wieder in der Ansaugphase ist.

Möglich ist weiter, dass der flexible Bereich eine umlaufend konkav ausgebildete Seitenwandung aufweist, die einen sich zum Bereich mit dem Permanentmagneten und/oder dem permanentmagnetischen Bereich verringernden Durchmesser aufweist. Dies kann vorteilhafterweise dadurch realisiert werden, dass der flexible Bereich eine im wesentlichen rund ausgeführte, domartige Pumpkammer einer Membranpumpe ist. In diesem Fall ist der Bereich mit dem Permanentmagneten und/oder dem permanentmagnetischen Bereich im oberen Bereich der Kammer angeordnet, wenn man die Wandung, die beispielsweise durch den zweiten Teil des Einwegelements ausgebildet wird, als Kammerboden betrachtet.

Es kann vorteilhafterweise vorgesehen sein, dass die Seitenwandung federt und einem Eindrücken des flexiblen Bereichs eine Federkraft entgegensetzt, die bei Entlastung eine Selbstrückstellung des flexiblen Bereiches bewirkt. Der flexible Bereich bzw. die Membran der Membranpumpe kann also derart ausgestaltet sein, dass sich beim Aktivieren aus ihrer Ruhelage in der Membran, bzw. in den Seitenwandungen der Membran, eine Materialspannung aufbaut, so dass sich gleichzeitig eine Rückstellkraft einstellt. Durch das Anlegen des Magnetfeldes muss also, um eine Pumpfunktion zu erzeugen, die Membran nur eingedrückt werden, so dass ein Magnetfeld nur in einer einzigen Auslenkungsrichtung angelegt werden muss. Die entsprechend gegenläufige Bewegung zur Rückstellung erfolgt sodann alleine aus der Rückstellkraft des flexiblen Bereiches bzw. der Membran. Ein Umpolen des Magnetfeldes ist somit nicht erforderlich.

Die Rückwärtsbewegung der Membran kann aber ebenfalls, wie bereits vorstehend beschrieben, durch ein umgekehrtes Magnetfeld unterstützt werden. Dies ist jedoch vorzugsweise nur dann möglich, wenn in der Membran ein Permanentmagnet als magnetisches Element verwendet wird. Die Membran kann somit nur von einer Seite kontinuierlich mit homogenem Kraftprofil betrieben werden.

Des Weiteren betrifft die vorliegende Erfindung ein System zum Pumpen mit den Merkmalen des Anspruchs 9. Danach ist vorgesehen, dass ein System zum Pumpen ein Einwegelement gemäß einem der Ansprüche 1 bis 8 sowie eine Aufnahmevorrichtung, in die das Einwegelement einlegbar ist, aufweist. Dabei weist das System zumindest ein Mittel zur Erzeugung eines Magnetfeldes auf, mittels dessen der flexible Bereich des Einwegelements mit dem zumindest einen Permanentmagneten und/oder zumindest einen permanentmagnetischen Bereich bewegbar ist.

Darüber hinaus kann vorgesehen sein, dass die Aufnahmevorrichtung das Einwegelement formschlüssig umgreift und/oder dass das Mittel zur Erzeugung eines Magnetfeldes eine elektromagnetische Spule, vorzugsweise eine mit einem Eisenkern versehene Elektromagnetspule ist, wobei die Elektromagnetspule in einem Gehäuse mit einer Aufnahme für den flexiblen Bereich angeordnet ist.

Für die Ansteuerung des Elektromagneten im Wechselfeldbetrieb ist ein sinusförmiger Spannungsverlauf von Vorteil. Es können somit Spannungsspitzen vermieden werden, die eine Schädigung der Elektronik des Systems verursachen könnten. Andere Spannungsverläufe sind ebenfalls denkbar, sofern eine verbesserte Pumpleistung erzielt werden soll.

Die Pumpbewegung der Membran ist vorteilhafterweise mit weiteren funktionellen Teilen der Maschinenseite oder des Einwegelements korreliert. Insbesondere kann die Membranposition mit Ventileinheiten auf dem Einwegelement geregelt werden. Nach einer entsprechenden Anordnung können so Flüssigkeiten gepumpt oder gesaugt werden.

Weiter ergibt sich durch eine elektromagnetische Ansteuerung der Vorteil, dass das System in der Lage ist, die Pumprate zu steuern. Die Pumprate kann auch durch eine elektrische Regelung variiert werden und auf Anweisungen einer entsprechenden Steuereinheit reagieren. In diesem Zusammenhang ist denkbar, dass das System über entsprechende Regelungs- und/oder Steuermittel verfügt. Weiter ist denkbar, dass über eine Kalibrierung bzw. aufgrund vorbekannter Volumenverhältnisse und anhand der elektrischen Ansteuerung Rückschlüsse auf die Pumpleistung gezogen werden können. Dadurch wird es möglich, eine hochgenaue Bilanzierung des geförderten Flüssigkeitsvolumens zu erreichen.

Besonders vorteilhaft ist es, wenn das System eine Blutbehandlungsvorrichtung, insbesondere eine Dialysemaschine ist. Ebenso kann vorgesehen sein, dass es sich um eine Analyseeinheit handelt.

Des Weiteren betrifft die vorliegende Erfindung ein Verfahren zum Pumpen einer Flüssigkeit mit den Merkmalen des Anspruchs 12. Danach ist vorgesehen, dass bei einem Verfahren zum Pumpen einer Flüssigkeit durch Anlegen eines Magnetfeldes ein flexibler Bereich mit zumindest einem Permanentmagneten und/oder mit zumindest einem permanentmagnetischen Bereich eines Einwegelementes die Flüssigkeit verdrängt wird. Dabei besteht das Einwegelement aus zumindest einem ersten Teil, in welchem in der Oberfläche Kanalstrukturen ausgenommen sind, und einem dieses dichtend abdeckenden zweiten Teil, wobei das erste Teil und/oder das zweite Teil zum größten Teil starr ausgebildet und/oder auf einer starren Trägerstruktur aufgebracht sind und wobei diese ersten und/oder zweiten Teile jedoch zumindest einen flexibel ausgebildeten Bereich aufweisen.

Erfindungsgemäß wird das Verfahren mit einem Einwegelement nach einem der Ansprüche 1 bis 8 und/oder einem System nach einem der Ansprüche 9 bis 11 durchgeführt .

Weitere Einzelheiten und Vorteile der Erfindung sollen nun anhand eines in der Zeichnung dargestellten Ausführungsbeispiels näher erläutert werden. Es zeigt:

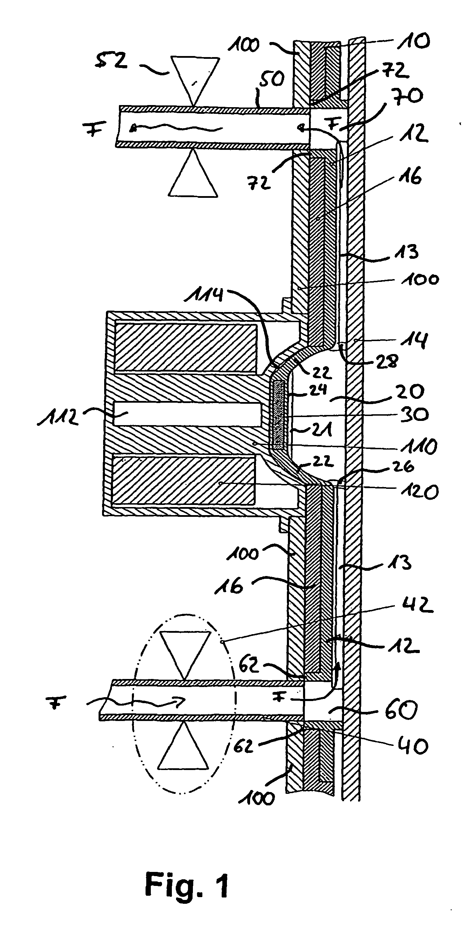

- Figur 1:: eine schematische Schnittdarstellung durch ein System zum Pumpen von Flüssigkeiten.

Figur 1 zeigt im Schnitt ein erfindungsgemäßes System zum Pumpen von Flüssigkeiten, wobei ein Einwegelement 10 in die Aufnahmevorrichtung des ansonsten nur durch das Gehäuse 110 mit einer Aufnahme 114 und der Elektromagnetspule 120 dargestellten Systems aufgenommen ist.

Das Einwegelement 10 weist einen ersten Teil 12 auf, der als flexible Lage 12 ausgebildet ist. In dem flexiblen ersten Teil 12 sind Kanalstrukturen 13 ausgenommen, die vom zweiten Teil 14, der als starrer Kassettenboden 14 ausgeführt ist, abgedichtet werden. Das flexible erste Teil 12 wird durch eine starre Trägerstruktur 16 getragen, so dass die flexible Lage 12 zwischen dem Trägerelement 16 und dem Boden 14 eingefasst ist. Das Einwegelement 10 ist dabei teilweise im Zweikomponentenspritzgussverfahren gefertigt, wobei das flexible erste Teil 12 und die starre Trägerstruktur 16 im Zweikomponentenspritzgussverfahren gefertigt werden. Der Boden 14 wird nachträglich aufgeklebt. Denkbar ist auch, dass der Boden 14 durch eine Folie ausgebildet wird.

Das Einwegelement 10 ist dabei als Einwegkassette 10 ausgeführt.

Im mittleren Bereich der Einwegkassette 10 befindet sich die Pumpkammer 20 einer elektromagnetisch betätigten Membranpumpe. Die Pumpmembran 21 ist ein flexibler Bereich 21, der einen Teil des ersten Teils bzw. der flexiblen ersten Lage 12 ist. Der flexible Bereich 21 erhebt sich dabei domartig aus der Lage 12 und durchdringt die Trägerstruktur 16. Die Pumpmembran 21 weist aus flexiblem Elastomer ausgebildete Seitenwandungen 22 auf, die vorzugsweise als umlaufend kreisförmig durchgängige Seitenwandung 22 ausgebildet sein können. Die Pumpkammer 20 ist in einem derartigen Fall rund, vorzugsweise kreisrund ausgebildet. Geht man davon aus, dass der Kammerboden durch die Lage 14 bzw. den zweiten Teil 14 des Einwegelements 10 ausgebildet ist, so ist das Pumpkammerdach durch den Wandungsbereich 24 des flexiblen Bereichs 21 ausgebildet.

Grundsätzlich kann die Einwegkassette 10 mit der Pumpkammer 20 beliebig ausgerichtet sein, also etwa in senkrechter, waagrechter oder schräger Ausrichtung ein eine entsprechende Aufnahme eingesetzt und betrieben werden. Die Funktionalität der Membranpumpe wird hierdurch nicht beeinträchtigt.

In diesem Wandungsbereich 24 befindet sich der Permanentmagnet 30, der vollständig vom flexiblen Bereich 21 ummantelt ist. Aufgrund der flexiblen Struktur und der domartigen Geometrie der Pumpmembran 21 ist diese selbstrückstellend, das heißt die Pumpmembran 21 kehrt im unbelasteten Zustand stets durch Rückfedern in die in Figur 1 dargestellte Nullstellung zurück.

Die Pumpkammer 20 weist einen Einlauf 26 und einen Auslauf 28 auf, die jeweils am Ende einer zuführenden bzw. abführenden Kanalstruktur 13 angeordnet sind. Die zuführende Kanalstruktur 13 kommt vom Kassetteneinlauf 60, der mittels eines Dichtelementes 62 an eine Fluidzuführung 40 angeschlossen werden kann. Das Dichtelement 62 ist dabei durch einen Ausläufer, vorzugsweise ringförmigen Ausläufer der flexiblen Lage 12 ausgeformt, die den Träger 16 durchdringt. Der Fluidzulauf 40 weist weiter ein Ventilelement 42 auf, mittels dessen der Zulauf gesperrt werden kann. Die Richtung des einströmenden Fluids F ist durch Pfeile angedeutet.

Der Kassettenablauf 70 ist im Wesentlichen baugleich dem Einlauf 60 ausgebildet und weist ebenfalls ein Dichtelement 72 auf, das ringförmig durch die flexible Lage 12 ausgebildet ist. Der Fluidablauf 70 ist dabei mit dem Ablaufkanal 50 verbindbar, wobei der Ablaufkanal 50 ein Ventilelement 52 aufweist, mittels dessen der Ablaufkanal 50 sperrbar ist.

Maschinenseitig ist eine Auflagefläche 100 vorgesehen, auf der die Trägerstruktur 16 aufgelegt werden kann. Die Auflagefläche 100 wird vom Gehäuse 110 des Mittels zur Erzeugung eines Magnetfeldes durchbrochen. Das Mittel zur Erzeugung eines Magnetfeldes ist eine Spule 120, die vollständig vom Gehäuse umgriffen wird. Weiter weist das Gehäuse 110 eine Aufnahme 114 auf, die an die Geometrie der Pumpmembran 21 angepasst ist. Weiter weist das Gehäuse 110 einen Hohlraum 112 auf, in den gegebenenfalls ein Eisenkern zur Verstärkung des Magnetfeldes eingelegt werden kann.

Die Funktionsweise des in Figur 1 dargestellten Systems zum Pumpen kann dabei wie folgt ausgestaltet sein: Mittels des Elektromagneten 120 wird ein Magnetfeld angelegt, so dass der Permanentmagnet 30 abgestoßen und in Richtung der Wandung 14 getrieben wird. Zugleich muss das Ventilelement 42 geschlossen sein, um eine Rückstrombewegung zu verhindern, und es muss das Ventilelement 52 geöffnet sein, um einen Abfluss des Fluids F zu ermöglichen. Durch Abschalten des Magnetfeldes bzw. des Elektromagneten 120 stellt sich die Pumpmembran 21 selbsttätig zurück. Vorteilhafterweise ist im Moment des Zurückstellens bereits das Ventilelement 52 geschlossen und das Ventilelement 42 geöffnet, so dass Fluid F erneut in die Pumpkammer 20 nachströmen kann. Sodann wird erneut das Ventil 42 geschlossen und das Ventil 52 geöffnet und durch den Elektromagneten 120 das Magnetfeld angelegt, so dass durch das Abstoßen des Permanentmagneten 30 erneut eine Verdrängung von Fluid aus der Pumpkammer 20 stattfindet.

## Patentansprüche

1. Einwegelement (10) bestehend aus zumindest einem ersten Teil (12), in welchem in der Oberfläche Kanalstrukturen (13) ausgenommen sind, und einem dieses dichtend abdeckenden zweiten Teil (14), wobei das erste Teil (12) und/oder das zweite Teil (14) zum größten Teil starr ausgebildet und/oder auf einer starren Trägerstruktur (16) aufgebracht ist/sind und wobei diese ersten und/oder zweiten Teile (12, 14) jedoch zumindest einen flexibel ausgebildeten Bereich (21) aufweisen, wobei der wenigstens eine flexible Bereich (21) zumindest einen Permanentmagneten (30) und/oder zumindest einen permanentmagnetischen Bereich aufweist, so dass durch Anlegen eines Magnetfeldes mittels des flexiblen Bereichs (21) Flüssigkeit verdrängbar ist
**dadurch gekennzeichnet,**
**dass** das Einwegelement (10) hergestellt ist durch ein Verfahren, in welchem starre und flexible Bereiche des ersten und/oder zweiten Teils (12, 14) mittels Zweikomponentenspritzguss einstückig hergestellt werden, wobei der Permanentmagnet (30) und/oder der permanentmagnetische Bereich ein Einlegeteil ist, dass vollständig vom dem flexiblen Bereich (21) ummantelt wird.

2. Einwegelement (10) nach Anspruch 1, **dadurch gekennzeichnet, dass** Trägerstruktur (16) und/oder erstes und/oder zweites Teil (12, 14) mittels Zweikomponentenspritzguss einstückig hergestellt werden.

3. Einwegelement (10) nach einem der vorhergehenden Ansprüche, **dadurch gekennzeichnet, dass** der wenigstens eine flexible Bereich (21) mit dem Permanentmagnet (30) und/oder dem permanentmagnetischen Bereich kammerartig und/oder domartig ausgebildet ist.

4. Einwegelement (10) nach Anspruch 3, **dadurch gekennzeichnet, dass** der kammerartig ausgebildete flexible Bereich (21) eine Pumpkammer (20) einer Membranpumpe ist.

5. Einwegelement (10) nach einem der vorhergehenden Ansprüche, **dadurch gekennzeichnet, dass** der flexible Bereich (21) zumindest einen Zulauf (26) und zumindest einen Ablauf (28) aufweist, wobei Zulauf (26) und Ablauf (28) jeweils durch eine Kanalstruktur (13) gebildet werden und/oder an eine Kanalstruktur (13) angeschlossen sind.

6. Einwegelement (10) nach einem der Ansprüche 4 oder 5, **dadurch gekennzeichnet, dass** der Permanentmagnet (30) und/oder der permanentmagnetische Bereich in einem Teil des flexiblen Bereichs (21) angeordnet ist, der einer Wandung gegenüberliegt, so dass durch Anlegen eines Magnetfeldes mit einer ersten Ausrichtung der flexible Bereich (21) in Richtung der Wandung bewegbar ist und/oder dass durch Anlegen eines Magnetfeldes mit einer zweiten Ausrichtung der flexible Bereich (21) von der Wandung weg bewegbar ist.

7. Einwegelement (10) nach einem der vorhergehenden Ansprüche, **dadurch gekennzeichnet, dass** der flexible Bereich (21) eine umlaufend konkav ausgebildete Seitenwandung (22) aufweist, die einen sich zum Bereich (24) mit dem Permanentmagneten (30) und/oder dem permanentmagnetischen Bereich verringernden Durchmesser aufweist.

8. Einwegelement (10) nach Anspruch 7, **dadurch gekennzeichnet, dass** die Seitenwandung (22) federt und einem Eindrücken des flexiblen Bereichs (21) eine Federkraft entgegensetzt, die bei Entlastung eine Selbstrückstellung des flexiblen Bereiches (21) bewirkt.

9. System zum Pumpen umfassend ein Einwegelement (10) gemäß einem der Ansprüche 1 bis 8 sowie eine Aufnahmevorrichtung, in die das Einwegelement (10) einlegbar ist, wobei das System zumindest ein Mittel zur Erzeugung eines Magnetfeldes aufweist, mittels dessen der flexible Bereich (21) des Einwegelements (10) mit dem zumindest einen Permanentmagneten (30) und/oder zumindest einen permanentmagnetischen Bereich bewegbar ist.

10. System nach Anspruch 9, **dadurch gekennzeichnet, dass** die Aufnahmevorrichtung das Einwegelement (10) formschlüssig umgreift und/oder dass das Mittel zur Erzeugung eines Magnetfeldes eine Elektromagnetspule (120), vorzugsweise eine mit einem Eisenkern versehene Elektromagnetspule (120) ist, wobei die Elektromagnetspule (120) in einem Gehäuse (110) mit einer Aufnahme (114) für den flexiblen Bereich (21) angeordnet ist.

11. System nach Anspruch 9 oder 10, **dadurch gekennzeichnet**, **dadurch gekennzeichnet, dass** das System eine Blutbehandlungsvorrichtung, insbesondere eine Dialysemaschine, und/oder ein Analysesystem ist.

12. Verfahren zum Pumpen einer Flüssigkeit, insbesondere einer medizinischen Flüssigkeit, mit einem Einwegelement nach einem der Ansprüche 1 bis 8 und/oder einem System nach einem der Ansprüche 9 bis 11, **dadurch gekennzeichnet, dass** durch Anlegen eines Magnetfeldes ein flexibler Bereich (21) mit zumindest einem Permanentmagneten (30) und/oder mit zumindest einem permanentmagnetischen Bereich eines Einwegelementes (10) die Flüssigkeit verdrängt wird, wobei das Einwegelement (10) aus zumindest einem ersten Teil (12), in welchem in der Oberfläche Kanalstrukturen (13) ausgenommen sind, und einem dieses dichtend abdeckenden zweiten Teil (14) besteht, wobei das erste Teil (12) und/oder das zweite Teil (14) zum größten Teil starr ausgebildet und/oder auf einer starren Trägerstruktur (16) aufgebracht sind und wobei diese ersten und/oder zweiten Teile (12, 14) jedoch zumindest einen flexibel ausgebildeten Bereich (21) aufweisen.

## Claims

1. A disposable element (10) comprising at least one first part (12) in which channel structures (13) are recessed in the surface and a second part (14) sealingly covering it, wherein the larger part of the first part (12) and/or of the second part (14) is made rigid and/or is applied to a rigid carrier structure (16) and wherein these first and/or second parts (12, 14), however, have at least one region (21) made flexible, wherein the at least one flexible region (21) has at least one permanent magnet (30) and/or at least one permanent magnetic region so that liquid can be displaced by means of the flexible region (21) by application of a magnetic field,
**characterized in that**
the disposable element (10) is manufactured by a method in which rigid and flexible regions of the first and/or second part (12, 14) are made in one piece by means of two-component injection molding, wherein the permanent magnet (30) and/or the permanent magnetic region is an insertion part which is completely encased by the flexible region (21).

2. A disposable element (10) in accordance with claim 1, **characterized in that** the carrier structure (16) and/or first and/or second parts (12, 14) are made in one piece by means of two-component injection molding.

3. A disposable element (10) in accordance with one of the preceding claims, **characterized in that** the at least one flexible region (21) is made in chamber-like form and/or in dome-like form with the permanent magnet (30) and/or the permanent magnetic region.

4. A disposable element (10) in accordance with claim 3, **characterized in that** the flexible region (21) made in chamber-like form is a pump chamber (20) of a membrane pump.

5. A disposable element (10) in accordance with one of the preceding claims, **characterized in that** the flexible region (21) has at least one inflow (26) and at least one outflow (28), with the inflow (26) and the outflow (28) each being formed by a channel structure (13) and/or being connected to a channel structure (13).

6. A disposable element (10) in accordance with one of the claims 4 or 5, **characterized in that** the permanent magnet (30) and/or the permanent magnetic region is arranged in a part of the flexible region (21) which is disposed opposite a wall so that the flexible region (21) is movable in the direction of the wall by application of a magnetic field with a first orientation; and/or **in that** the flexible region (21) is movable away from the wall by application of a magnetic field with a second orientation.

7. A disposable element (10) in accordance with one of the preceding claims, **characterized in that** the flexible region (21) has a peripherally concavely made side wall (22) which has a diameter reducing toward the region (24) with the permanent magnet (30) and/or the permanent magnetic region.

8. A disposable element (10) in accordance with claim 7, **characterized in that** the side wall (22) is resilient and opposes a pressing in of the flexible region (21) with a resilient force which effects a self-restoration of the flexible region (21) on load removal.

9. A system for pumping comprising a disposable element (10) in accordance with one of the claims 1 to 8 as well as a reception apparatus into which the disposable element (10) can be inserted, with the system having at least one means for the generation of a magnetic field by means of which the flexible region (21) of the disposable element (10) with the at least one permanent magnet (30) and/or at least one permanent magnetic region is movable.

10. A system in accordance with claim 9, **characterized in that** the receiving apparatus engages around the disposable element (10) in a shape-matched manner; and/or **in that** the means for the generation of a magnetic field is an electromagnetic coil (120), preferably an electromagnetic coil (120) provided with an iron core, with the electromagnetic coil (120) being arranged in a housing (110) having a receiver (114) for the flexible region (21).

11. A system in accordance with either of claims 9 or 10, **characterized in that** the system is a blood treatment apparatus, in particular a dialysis machine and/or an analysis system.

12. A method for the pumping of a liquid, in particular of a medical liquid, having a disposable element in accordance with one of the claims 1 to 8 and/or a system in accordance with one of the claims 9 to 11, **characterized in that** a flexible region (21) with at least one permanent magnet (30) and/or with at least one permanent magnetic region of a disposable element (10) displaces the liquid by application of a magnetic field, with the disposable element (10) being made from at least one first part (12) in which channel structures (13) are recessed in the surface and from a second part (14) sealingly covering it, with the larger part of the first part (12) and/or of the second part (14) being made rigid and/or being applied to a rigid carrier structure (16), and with these first and/or second parts (12, 14), however, having at least one region (21) made flexible.

## Revendications

1. Élément à usage unique (10) se composant d'au moins une première partie (12), dans laquelle des structures de canaux (13) sont creusées dans la surface, et d'une seconde partie (14) recouvrant celle-ci hermétiquement, la première partie (12) et/ou la seconde partie (14) étant constituée(s) de manière rigide pour la plus grande partie et/ou appliquée(s) sur une structure de support rigide (16) et ces première et/ou seconde parties (12, 14) présentant toutefois au moins une zone constituée de manière flexible (21), au moins la zone flexible (21) présentant au moins un aimant permanent (30) et/ou au moins une zone magnétique permanente, de sorte que, par l'établissement d'un champ magnétique au moyen de la zone flexible (21), du liquide est refoulable, **caractérisé en ce que**
l'élément à usage unique (10) est fabriqué par un procédé dans lequel des zones rigides et flexibles de la première et/ou seconde partie (12, 14) sont fabriquées en une pièce au moyen d'un moulage par injection bicomposantes, l'aimant permanent (30) et/ou la zone magnétique permanente étant une pièce d'insertion, qui est enrobé entièrement par la zone flexible (21).

2. Élément à usage unique (10) selon la revendication 1, **caractérisé en ce que** la structure de support rigide (16) et/ou les première et/ou seconde parties (12, 14) sont fabriquées en une pièce au moyen d'un moulage par injection bicomposantes.

3. Élément à usage unique (10) selon une quelconque des revendications précédentes, **caractérisé en ce qu'**au moins la zone flexible (21) est constituée en chambre et/ou en dôme avec l'aimant permanent (30) et/ou la zone magnétique permanente.

4. Élément à usage unique (10) selon la revendication 3, **caractérisé en ce que** la zone flexible (21) constituée en chambre est une chambre de pompe (20) d'une pompe à membrane.

5. Élément à usage unique (10) selon une quelconque des revendications précédentes, **caractérisé en ce que** la zone flexible (21) présente au moins une alimentation (26) et au moins une sortie (28), l'alimentation (26) et la sortie (28) étant constituées respectivement par une structure de canaux (13) et/ou raccordées à une structure de canaux (13).

6. Élément à usage unique (10) selon une quelconque des revendications 4 ou 5, **caractérisé en ce que** l'aimant permanent (30) et/ou la zone magnétique permanente est disposé dans une partie de la zone flexible (21) qui se trouve face à une paroi de sorte que par l'établissement d'un champ magnétique avec un premier alignement, la zone flexible (21) est mobile en direction de la paroi et/ou par l'établissement d'un champ magnétique avec un deuxième alignement, la zone flexible (21) est mobile en s'éloignant de la paroi.

7. Élément à usage unique (10) selon une quelconque des revendications précédentes, **caractérisé en ce que** la zone flexible (21) présente une paroi latérale constituée de manière concave sur le pourtour (22), qui présente un diamètre se réduisant vers la zone (24) avec l'aimant permanent (30) et/ou la zone magnétique permanente.

8. Élément à usage unique (10) selon la revendication 7, **caractérisé en ce que** la paroi latérale (22) rebondit et oppose une élasticité à un enfoncement de la zone flexible (21), qui en cas de relâchement, entraîne un retour à l'état initial de la zone flexible (21).

9. Système de pompage comportant un élément à usage unique (10) selon une quelconque des revendications 1 à 8, ainsi qu'un dispositif de réception, dans lequel l'élément à usage unique (10) est introduisible, le système présentant au moins un moyen destiné à la production d'un champ magnétique au moyen duquel la zone flexible (21) de l'élément à usage unique (10) est mobile au moins avec un aimant permanent (30) et/ou au moins une zone magnétique permanente.

10. Système selon la revendication 9, **caractérisé en ce que** le dispositif de réception entoure mécaniquement l'élément à usage unique (10) et/ou **en ce que** le moyen destiné à la production d'un champ magnétique est une bobine d'électroaimant (120), de préférence une bobine d'électroaimant (120 dotée d'un noyau de fer, la bobine d'électroaimant (120) étant disposée dans un boîtier (110) avec une réception (114) pour la zone flexible (21).

11. Système selon la revendication 9, **caractérisé en ce que** le système est un dispositif de traitement sanguin, notamment une machine de dialyse et/ou un système d'analyse.

12. Procédé destiné au pompage d'un liquide, notamment d'un liquide médical, avec un élément à usage unique selon une quelconque des revendications 1 à 8 et/ou un système selon une quelconque des revendications 9 à 11, **caractérisé en ce que**, par l'établissement d'un champ magnétique au moyen d'une zone flexible (21) avec au moins un aimant permanent (30) et/ou au moins une zone magnétique permanente d'un élément à usage unique (10), le liquide est refoulé, l'élément à usage unique (10) se composant d'au moins une première partie (12), dans laquelle des structures de canaux (13) sont creusées dans la surface, et d'une seconde partie (14) recouvrant celle-ci hermétiquement, la première partie (12) et/ou la seconde partie (14) étant constituée(s) de manière rigide pour la plus grande partie et/ou appliquée(s) sur une structure de support rigide (16) et ces première et/ou seconde parties (12, 14) présentant toutefois au moins une zone constituée de manière flexible (21).
